# EUROPÄISCHE PATENTSCHRIFT

(11) **EP 2 137 203 B1**
(45) Veröffentlichungstag und Bekanntmachung des Hinweises auf die Patenterteilung: **30.09.2015**
(21) Anmeldenummer: 08736044.2
(22) Anmeldetag: 10.04.2008
(51) Int. Cl.: C07H 21/00, A61K 31/7084, A61P 35/00

(54) **ETHINYLIERTE HETERODINUCLEOSIDPHOSPHATANALOGA, VERFAHREN ZU DEREN HERSTELLUNG UND DEREN VERWENDUNG**
ETHYNYLATED HETERODINUCLEOSIDE PHOSPHATE ANALOGS, METHOD FOR THE PRODUCTION THEREOF, AND USE THEREOF
ANALOGUES D'HÉTÉRODINUCLÉOSIDE PHOSPHATE ETHYNYLATÉS, PROCÉDÉ DE PRODUCTION ET UTILISATION DE CES DERNIERS

(30) Priorität: 13.04.2007 EP 07007635
(43) Veröffentlichungstag der Anmeldung: 30.12.2009
(73) Patentinhaber: SCHOTT, Herbert, 72076 Tübingen (DE)
(72) Erfinder: SCHOTT, Herbert, 72076 Tübingen (DE); LUDWIG, Peter, 72764 Reutlingen (DE)
(74) Vertreter: Reitstötter Kinzebach
(86) Internationale Anmeldenummer: PCT/EP2008/054322
(87) Internationale Veröffentlichungsnummer: WO 2008/125583

(56) Entgegenhaltungen:
- LUDWIG, PETER S. ET AL.: "Synthesis and anticancer activities of amphiphilic 5-fluoro-2'-deoxyuridylic acid prodrugs" EUROPEAN JOURNAL OF MEDICINAL CHEMISTRY, Bd. 40, 2005, Seiten 494-504, XP002441281 in der Anmeldung erwähnt
- LUDWIG, PETER S. ET AL.: "A new laboratory scale synthesis for the anticancer drug 3'-C-ethynylcytidine" SYNTHESIS, Nr. 16, 2002, Seiten 2387-2391, XP002441282 in der Anmeldung erwähnt
- MARTY, CORNELIA ET AL: "Isolation and characterization of a scFv antibody specific for tumor endothelial marker 1 (TEM1), a new reagent for targeted tumor therapy" CANCER LETTERS (AMSTERDAM, NETHERLANDS) , 235(2), 298-308 CODEN: CALEDQ; ISSN: 0304-3835, 2006, XP002441280
- BUFF, ROLF ET AL: "2'-Ethynyl-DNA: synthesis and pairing properties" HELVETICA CHIMICA ACTA ; ISSN: 0018-019X, Bd. 85, Nr. 1, 2002, Seiten 224-254, XP002486676

## Beschreibung

Die vorliegende Erfindung betrifft neue Wirkstoffe, deren Herstellung, Mittel enthaltend wenigstens eine dieser Verbindungen sowie deren Verwendung zur Behandlung von Krebserkrankungen und Infektionskrankheiten.

### Hintergrund der Erfindung

Nucleosidanaloga, die bestimmte Strukturmerkmale aufweisen, sind bewährte Arzneimittel in der Chemotherapie von Krebserkrankungen und virusbedingten Krankheiten (Advanced Drug Delivery Review (1996)19,287). Analoga des Cytidins, wie z.B. 1ß-D-Arabinofuranosylcytosin (araC), oder des Uridins, wie z.B. 5-Fluoro-2-deoxyuridin (5FdU), verhindern die DNA-Replikation und wirken gegen maligne Erkrankungen der blutbildenden Zellen und gegen solide Tumore. Zur Therapie der HIV-Infektion eignen sich besonders Didesoxynucleosidanaloga wie z.B. 3'-Azido-2',3'-didesoxythymidin (AZT), 2',3'-Didesoxycytidin (ddC), 2',3'- Didesoxyinosin (ddl), 3'-Thia-2',3'didesoxycytidin (3TC) und 2',3'-Didehydro-2',3'-didesoxythymidin (d4T). Nucleo-sidanaloga mit Ethinylresten, wie z.B. 3'-C-Ethinylcytidin (ECyd), sind multifunktionelle Antitumor-Wirkstoffe mit einem breiten Aktivitätsspektrum (Hattori, H. et al. J.Med.Chem. 1996, 39, 5005; Azuma, A. et.al. Nucleosides, Nucleotides & Nucleic Acids, 2001, 20. 609)

Die therapeutische Wirkung von Nucleosidanaloga setzt voraus, dass die applizierten Nucleosidanaloga, die in der Regel unwirksame "Prodrugs" sind, von der Zelle aufgenommen und zu den eigentlichen Wirkstoffen, den 5'-Triphosphatderivaten der Nucleosidanaloga anabolisiert werden. Die phosphorylierten Derivate können die DNA-und/oder RNA-Synthese mit lethalen Folgen für die Zelle beeinträchtigen oder die Virusvermehrung unterbinden.

Aufgrund der häufig auftretenden Resistenzbildung im Verlauf der Chemotherapie mit nur einem Wirkstoffe (Monotherapie) kann der applizierte Arzneistoff im Laufe der Therapie seine Wirkung verlieren. Um die Progression der Erkrankung nachhaltig zu verlangsamen und um der Resistenzbildung effektiv entgegenzuwirken, werden verschiedene Wirkstoffe gemeinsam (Kombinationstherapie) appliziert. Die Applikation einer Darreichungsform zur HIV Therapie (Schweiz. Med. Wochenschr. (1997), 127, 436) in der z.B. AZT und 3TC als Gemisch vorliegen, wie dies bei "Combivir" der Fall ist, macht die Kombinationstherapie für den Patienten allenfalls nur praktikabler. Eine verbesserte antivirale Wirkung ist aber mit solchen Gemischen kaum erreichbar, da weder die Zellaufnahme der als Gemisch applizierten Wirkstoffe gesteigert, noch deren Anabolisierung in die entsprechenden Triphosphatderivate optimiert wird.

Dagegen lässt sich die antivirale und/oder cytostatische Wirkung von Kombinationspräparaten erheblich optimieren, wenn die unterschiedlichen Nucleosidanaloga in einer Darreichungsform chemisch gekuppelt sind. Ausschlaggebend für die therapeutische Wirkung solcher Kombinationspräparate ist die jeweils gewählte Art der Verknüpfung der beiden monomeren Wirkstoffe zu einem neuen Kombinationswirkstoff. Die kovalente Kupplung muss gewährleisten, dass bei einer gewünschten Metabolisierung therapeutisch wirksame Metabolite freigesetzt werden können, die additive oder gar synergistische Effekte auslösen und möglicherweise Resistenzmechanismen gegen die monomeren Wirkstoffe aufheben.

Bei der Kupplung eines lipophilen mit einem hydrophilen Nucleosidanaloga über eine Phosphodiesterbrücke resultieren amphiphile Dinucleosidphosphatanaloga (EP-A-0642527). Die Verknüpfung unterschiedlicher hydrophiler Nucleosidanaloga über ein Glycerinlipidgerüst führt zu amphiphilen Glycerylnucleotiden (DE-A-19855963 oder WO-A-00/34298). Die in diesen Kombinationspräparaten gewählte Art der Kupplung erfüllt die gestellte Anforderung. Beide Kombinationspräparate tragen erheblich zur Verbesserung der Chemotherapie von Tumor- und Viruserkrankungen bei.

Das breite Aktivitätssprektrum von Nucleosidanaloga, die einen Ethinylrest aufweisen, wird bisher nur auf der Basis der Glycerylnucleotidanaloga genutzt. Ein deutlicher Nachteil dieser Art von Kombinationspräparaten ist ihr hoher Syntheseaufwand, der vor allem durch die mehrstufige Synthese des Glyceryllipidgerüstes bedingt wird. Außerdem kann das resultierende hohe Molgewicht die Verteilung dieser Kombinationspräparate und damit verbunden das Wirkstofftargeting in vivo erschweren, wodurch die therapeutische Wirkung beeinträchtigt werden kann.

Aus Ludwig, P.S. et al., European Journal of Medical Chemistry 2005 494-504 sind nicht-ethinylierte 3'-5' und 5'-5'- verknüpfte Duplexwirkstoffe mit Antitumoraktivität bekannt.

Inwieweit sich eine unnatürliche (d.h. 5'-5') und insbesondere aber eine natürliche (d.h. 3'-5') Phosphodiesterbrücke zur Verknüpfung von ethinylierten Nucleosidanaloga mit anderen therapeutisch wirksamen Verbindung auf Nucleosidbasis eignen, ist weitgehend ungeklärt, da der Einfluss eines Ethinylrestes auf die Metabolisierung eines Dimeren ungeklärt ist. Es ist nicht auszuschließen, dass ein ethynyliertes Nucleosid am 3'-Ende des Dimeren die hydrolytische Entfernung des ethynylierten Bausteins verhindert, da das 3'-Ende durch den Ethinylrest für Exonucleasen maskiert ist, so dass die gewünschte Metabolisierung des Duplexwirkstoffs in die zwei monomeren Wirkstoffe nicht eintreten könnte.

Aus Marty, C., et al Cancer Letters 2006 , 235, 298-208 sind Immunoliposome bekannt, welche gegen den Tumormarker TEM1 gerichtet sind und dazu mit einem speziellen Antikörperfragment (ScFv-CM6) funktionalisiert sind. Zusätzlich wird vorgeschlagen, mit dem cytotoxischen Wirkstoff N⁴-Octadecyl-1-ß-D-arabinofuranosylcytosin-(5'-5')-3'-C-ethinylcytidin zu beladen. Ein derartiges Präparat ist aber dazu ausgelegt, den Wirkstoff direkt, d.h. ohne metabolische Spaltung, zum Tumor zu transportieren. Auf eine Eignung als klassisch, d.h, z.B. oral oder intraperitoneal, zu verabreichendes Medikament kann daraus noch nicht geschlossen werden.

Eine Syntheseroute zu Nucleosid-analogen 3'-C-Ethinylcytidinen ist aus Ludwig, P.S. et al., Synthesis 2002, 16, 2387 bekannt.

### Zusammenfassung der Erfindung

Aufgabe dieser Erfindung ist es, neue, leicht zugängliche Kombinationspräparate bereitzustellen, mit denen Krebs- und/oder Viruserkrankungen in neuartiger Weise behandelt werden können.

Die Aufgabe wird überraschenderweise durch neue Dinucleosidphosphatanaloga gelöst, die bei ihrer Metabolisierung gleichzeitig mehrere, verschieden aktive Nucleosidanaloga, wie z.B. mit unterschiedlichen Wirkmechanismen, freisetzen, von denen immer mindestens ein Nucleosidanaloga den therapeutisch hochwirksamen Ethinylrest trägt. Mit diesen so genannten Duplexwirkstoffen werden nicht nur die allgemeinen Vorteile einer Kombinationstherapie, sondern erstmals auch die multifunktionelle Wirksamkeit von ethinylierten Nucleosidanaloga in Kombinationspräparaten nutzbar. Der erforderliche, entscheidende Phosphorylierungsschritt zur Aktivierung der ethinylierten Nucleosidanaloga durch körpereigene Kinasen, wie die Uridin/Cytidin-Kinase, kann bei den Duplexwirkstoffen entfallen, da bei deren Metabolisierung das ethinylierte Nucleosid, wie z.B. Ecyd, bereits in der phosphorylierten Form gebildet werden kann. Hinzukommt, dass im Vergleich zu den ethinylierten Glycerylnucleotidanaloga der Syntheseaufwand für die Dinucleosidphosphatanaloga erheblich geringer ist.

Überraschenderweise zeigt außerdem eine erfindungsgemäß bevorzugte Klasse von ethinylierten Dinucleosidphosphatanaloga, die über eine natürliche 3'-5'-Phosphodiesterbrücke oder analoge End-Ring-Verknüpfungen verknüpft sind, und insbesondere solche, die das ethinylierte Monomer über dessen 5'- Position am 3'- Ende des nicht ethinylierten zweiten Monomers tragen, eine signifikant höhere AntitumorAktivität als die entsprechenden Isomere, die eine unnatürliche 5'-5'- Kupplung aufweisen. Die deutliche Überlegenheit der 3'-5'- Kupplung kann anhand der ermittelten Wirkstoffkonzentrationen für die totale Wachstumshemmung der Tumorzellen (siehe Beispiel 3, TGI Werte) gezeigt werden. Häufig liegen die TGI Werte für das 3'-5'- gekuppelte Isomer bis zu 1000-fach niedriger im Vergleich zu den Werten für das 5'-5'-gekuppelte Isomer. Die Art der Verknüpfung ist überraschenderweise für eine weiter verbesserte Wirksamkeit mit ausschlaggebend, da vermutlich bei der enzymatischen Metabolisierung sehr unterschiedlich aktive Wirkstoffe gebildet werden. Durch die Wahl der Richtung der Phosphodiesterbrücke lässt sich die Antitumoraktivität eines Kombinationspräparates überraschenderweise so modulieren, dass das Wirkspektrum der ethinylierten Duplexwirkstoffe weiter verbessert werden kann. Hinzu kommt noch, dass die natürliche 3'-5'-Verknüpfung gegenüber der unnatürlichen 5'-5'- Kupplung synthetisch wesentlich einfacher zugänglich ist und dann bevorzugt ist, wenn die Molekülstruktur der zur kuppelnden Monomere eine natürliche 3'-5'- Phosphodiesterbindung erlaubt.

### Detaillierte Beschreibung der Erfindung

### a) Allgemeine Begriffe

Werden keine anderen Angaben gemacht, so werden erfindungsgemäß sowohl einzelne Isomere erfindungsgemäßer Wirkstoffe als auch jegliche Mischungen stereoisomerer Formen davon mit umfasst. Insbesondere werden alle stereoisomeren Formen erfindungsgemäßer Verbindungen in Reinform sowie jegliche Mischungen solcher stereoisomerer Formen mit umfasst.

Weiterhin können erfindungsgemäße Dinucleosidphosphatanaloga beliebige Kombinationen von D- und L-Isomeren ihrer Nucleosidbausteine umfassen.

Weiterhin sind erfindungsgemäß alle möglichen diastereomeren oder anomeren Formen erfindungsgemäßer Verbindungen, insbesondere alpha- und beta-Anomere, mit umfasst.

Nucleosidreste umfassen erfindungsgemäß natürliche Nucleoside, wie Cytidinund/oder Uridin, die korrespondierende Mono- und Didesoxyformen und strukturanaloge Verbindungen, erhältlich durch Änderung des glycosidischen Rests und oder des Basenrests, wie unten näher erläutert.

Die terminale oder endständige Verknüpfung eines Nucleosids erfolgt gewöhnlich über eine HOCH₂- -Gruppe, eine ringständige Verknüpfung erfolgt gewöhnlich über eine -CH(OH)- Gruppe des glycosidischen Rests.

Erfindungsgemäße Verbindungen umfassen je nach Art der ggf. verwendeten Substituenten Verbindungen mit amphiphilen, lipophilen oder hydrophilen Charakter.

Eine erfindungsgemäße Behandlung einer Erkrankung umfasst sowohl die Prophylaxe als auch insbesondere die Therapie.

### b1) Erfindungsgemäße Heterodinucleosidphosphatanaloga

Gegenstand der Erfindung sind insbesondere ethinylierte Heterodinucleosidphosphatanaloga der Formel I 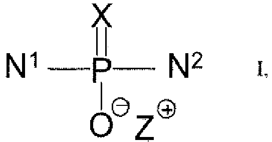 worin
X für O oder S steht;
Z für H oder das korrespondierende Säureadditionssalz dieser Verbindung steht;
N¹ für einen Nukleosidrest der Formeln IV 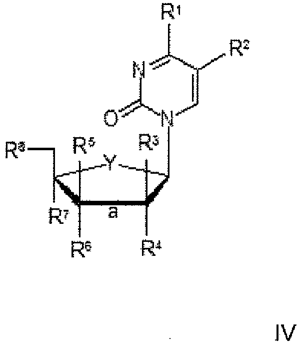 steht, der nicht ethyniliert ist, worin:
   R¹ für Hexadecyl-, Palmitoyl-, Oleoylamino oder Hydroxyl steht;
   R² für H oder Fluor steht;
   R³, R⁴ gleich oder verschieden sind und für H, Hydroxyl, Fluor steht;
   R⁵, R⁷ für H steht;
   R⁶ für ein Sauerstoffatom steht, mit dem N¹ mit P verbrückt ist,
   R⁸ für Hydroxyl, Azido oder H steht und
   Y für O oder S steht
   und N² für einen Nukleosidrest der Formel IV steht, der ethinyliert ist, worin
   R¹ für Amino
   R² ,R³ ,R⁷ für H;
   R⁴, R⁶ für Hydroxyl;
   R⁵ für Ethinyl;
   R⁸ für ein Sauerstoffatom steht, mit dem N² mit P verbrückt ist und
Y für O oder S steht. Insbesondere ist die Verknüpfung eine enzymatisch, insbesondere in vitro oder in vivo, im menschlichen Körper spaltbare Verknüpfung. Weiterhin kann die Verknüpfung der glycosidischen Reste terminal-terminal (End-End-Verknüpfung, wie z.B. 5'-5') oder terminal-ringständig (End-Ring-Verknüpfung, wie z.B. 3'-5') erfolgen. Insbesondere erfolgt die Verbrückung terminal-ringständig (End-Ring-Verknüpfung, wie z.B. 3'-5'). Eine "ringständige" Verknüpfung erfolgt dabei durch Verbrückung eines Ring-C-Atoms des glycosidischen oder davon abgeleiteten Rings des Nucleosids mit dem P-Atom, wie z.B. über das 3'-C-Atom einer Pentose. Eine "endständige" Verknüpfung erfolgt dabei durch Verbrückung eines endständigen, Nicht-Ring-C-Atoms des glycosidischen oder davon abgeleiteten Rings des Nucleosids mit dem P-Atom, wie z.B. über das terminale 5'-C-Atom einer Pentose. Dabei sind die Begriffe "End-Ring" und "Ring-End" als synonyme Begriffe zu verstehen, und nicht an eine bestimmte Reihenfolge gebunden. Entsprechendes gilt für die Begriffe "3'-5' " und "5'- 3' ", Auch ist die Positionierung des Ethinylsubstituenten nicht auf das End- oder Ring-verknüpfte Nucleosid festgelegt.

### b2) Definitionen allgemeiner Reste

Der glycosidische Rest des Nucleosids oder Nucleosidderivats ist abgeleitet von einer Pentose, wie z.B. Desoxyribose, Didesoxyribose oder Ribose. Im glycosidischen Rest können gegebenenfalls einzelne oder mehrere Protonen oder Hydroxylgruppen substituiert oder eliminiert sein. Geeignete Substituenten sind dabei ausgewählt unter Wasserstoff, F, Hydroxyl, Ethinyl

Der Basenteil des Nucleosids oder Nucleosidderivats ist der Rest einer sechsgliedrigen heterocyclische Base mit zwei Stickstoff-Heteroatomen, z.B. Cytosin und Uracil. Die genannten Basen können gegebenenfalls ein- oder mehrfach, wie z.B. ein- bis zweifach substituiert sein durch die oben angegebenen Reste Hydroxyl, Hexadecyl-, Palmitoyl- und/oder Oleoylamin. Dabei kann die Substitution bevorzugt an einem Ring-Kohlenstoffatom erfolgen.

### b3) Bevorzugte Verbindungen

Verbindungen, worin in N² für einen Nukleosidrest der Formel IV steht der ethinyliert ist, und N¹ für einen Nukleosidrest der Formel IV steht, der nicht ethinyliert ist, worin
R¹ für Hydroxyl steht;
R² für Fluor steht;
R³, R⁴, R⁵ und R⁷ für H stehen,
R⁶ für -O- steht, und
R⁸ für Hydroxyl steht.

Bevorzugte Klassen von Heterodinucleosidphosphatanaloga umfassen insbesondere als ethinylierte Komponente einen Nucleosidrest von einem 3' -C-Ethinylnucleosid, wie insbesondere von 3' -C-Ethinylcytidin.

Eine bevorzugte Einzelverbindung ist: 5-Fluoro-2'-desoxyuridylyl-(3'-5')-3'-C-ethinylcytidin

### c) Herstellung erfindungsgemäßer Heterodinucleosidphosphatanaloga

Die Erfindung betrifft außerdem Verfahren zur Herstellung von erfindungsgemäßen ethinylierten Heterodinucleosidphosphatanaloga, wobei man zwei Nucleoside der allgemeinen Formeln Va und Vb

L¹-N¹ (Va)

L²-N² (Vb)

worin
N¹ und N² wie oben definiert sind und gegebenenfalls eine oder mehrere Schutzgruppen aufweisen, wobei insbesondere wenigstens eine der Gruppen N¹ und N² am glycosidischen Rest eine Ethinyl- oder geschützte Ethinyl-Gruppe trägt; und
L¹ und L² am glycosidischen Rest von N¹ und N² gebundene, miteinander reaktive Gruppen darstellen, wobei eine der Gruppen L¹ und L² für eine Hydroxy- oder Mercaptogruppe und der andere für eine Hydrogenphosphonat- oder Thiohydrogenphosphonatgruppe steht; und wobei insbesondere eine der Gruppen L¹ und L² ringständig und die andere endständig gebunden ist;
in Gegenwart eines Säurechlorids kondensiert und das Kondensationsprodukt anschließend oxidiert, insbesondere um die bei der Kondensation entstandene Phosphonatbrücke zur Phosphatbrücke zu oxidieren, und gegebenenfalls vorhandene Schutzgruppen entfernt.

Eine "ringständige" Bindung erfolgt dabei durch Bindung an ein Ring-C-Atom des glycosidischen oder davon abgeleiteten Rings des Nucleosids, wie z.B. mit dem 3'-C-Atom einer Pentose. Eine "endständige" Bindung erfolgt dabei durch Bindung an ein endständiges, Nicht-Ring-C-Atom des glycosidischen oder davon abgeleiteten Rings des Nucleosids, wie z.B. mit dem terminalen 5'-C-Atom einer Pentose.

Gegenstand ist insbesondere ein Herstellungsverfahren, wobei man jeweils zwei Nucleoside der Formeln Va und Vb umsetzt, wobei die Nucleoside Va und Vb einer Verbindung der obigen allgemeinen Formel II, III oder IV entsprechen, worin
X und a die oben angegebenen Bedeutungen besitzen,
L¹ und L² an Stelle eines der Reste R³ bis R⁸, insbesondere R⁶ oder R⁸, enthalten sind, die Reste R¹ bis R⁸ im übrigen die oben angegebene Bedeutung besitzen;
die Reste R¹ und R³ bis R⁸ zusätzlich auch für eine acylierte Hydroxygruppe, deren Acylrest linear oder verzweigt ist, 1-24 C-Atome und 1 oder 2 Doppelbindungen aufweist und durch einen aromatischen Rest substituiert sein kann, oder für tert-Butyldimethylsilyloxy-Schutzgruppe stehen können,
R⁸ zusätzlich auch für eine 4-Mono-, oder 4,4'-Dimethoxytriphenylmethyloxy-Schutzgruppe stehen kann;
und wenigstens einer der Reste R³ bis R⁸, insbesondere Rest R⁵ auch für Trimethylsilylethinyl stehen kann.

Weiterhin ist dabei bevorzugt, dass man gegebenenfalls vorhandene 4-Mono- oder 4,4'- Dimethoxytriphenylmethyloxy Schutzgruppen gegen Hydroxyl tauscht und Acyl- und Silylreste gegebenenfalls hydrolytisch spaltet.

Werden keine anderen Angaben gemacht, so erfolgt die Herstellung erfindungsgemäßer Verbindungen unter Anwendung an sich bekannter Verfahren oder Synthesetechniken die dem Fachmann auf dem Gebiet der Synthese von Nucleosidanalogen geläufig sind.

Die Kondensation gelingt besonders gut in Lösung in Gegenwart von Säureanhydriden oder Säurehalogeniden, wie insbesondere Pivalinsäurechlorid, bei -80 °C bis +100 °C, wie z.B. etwa 0 - 20°C. Ein geeignetes Lösungsmittel ist z.B. Pyridin.

Die Oxidation gelingt besonders gut in Lösung bei -80 °C bis +100 °C, wie z.B. etwa 0 - 20°C, wobei, a) mit Jod in wässrigen organischen Lösungsmitteln die P-H-Bindung zu einer P=O-Bindung oder b) mit S₈ in Triethylamin/CS₂ die P-H-Bindung zu einer P=S-Bindung oxidiert wird. Ein geeignetes Lösungsmittel ist z.B. THF.

Nach Oxidation und chromatographischer Aufarbeitung erfolgt die Abtrennung der Schutzgruppen in an sich bekannter Weise. Dabei wird beispielsweise die 4-Mono- oder 4,4'-Dimethoxytriphenylmethylgruppe gegen Hydroxyl, und/oder Trimethylsilyl gegen Wasserstoff getauscht und/oder Acylreste werden im Bedarfsfall hydrolytisch in Mercapto-, Hydroxyl- und/oder Aminogruppen überführt.

Die für die Umsetzungen benötigten Ausgangsmaterialien sind an sich bekannte Stoffe oder lassen sich in Analogie zu bekannten Verfahren herstellen (Antivir. Chem & Chemother, (1998) 9, 33; Makromol Chem. (1986) 187, 809; Tetrahedron Lett. (1986) 27, 2661; Synthesis (2002) 16, 2387; Eurp. J Med.Chem. (2005) 40, 494); worauf hiermit ausdrücklich Bezug genommen wird.

Insbesondere sind auch die erfindungsgemäß verwendeten ethinylierten Nucleosidbausteine an sich bekannt bzw. leicht herstellbar.(vgl. z.B. auch Bioorg. Med. Chem. (2005) 13, 2597-2621; Cancer Sci (2005), 96, 5, 295-302; J. Med. Chem. (1996) 39, 5005-5011; Radiation Research (2004) 162, 635-645); worauf hiermit ausdrücklich Bezug genommen wird.

Beispielsweise kann die Kondensation zweier Nucleosidederivate zum erfindungsgemäßen Duplexwirkstoff wie folgt durchgeführt werden:
Ein erstes, eine Hydrogenphosphonatgruppe tragendes geschütztes Nukleosidderivat (wie z.B. 5'-O-(4-Monomethoxytrityl)-5-fluoro-2'-desoxyuridin-3'-hydrogenphosphonat) wird zusammen mit einem zweiten eine geschützte Ethinylgruppe tragenden und gegebenenfalls weitere Schutzgruppen tragenden Nucleosidderivat (wie z.B. N⁴-Benzoyl-2'-O-(tert.-butyldimethylsilyl)-3'-C-(trimethylsilylethinyl)cytidin) in einem wasserfreien polaren Lösungsmittel, wie z.B. Pyridin, gelöst. Die auf ca. 0 bis 15°C abgekühlte Lösung wird unter Feuchtigkeitsausschluss mit einem geeigneten Kondensationshilfsmittel, wie einem Säurechlorid, wie z.B. Pivaloylchlorid, versetzt, nach Durchmischen und Umsetzung bei Raumtemperatur erneut abgekühlt und mit Wasser versetzt. Der Reaktionsansatz wird dann mit einer Lösung von Iod, z.B. in Tetrahydrofuran, versetzt und anschließend erneut bei Raumtemperatur umgesetzt. Überschüssiges Iod wird durch Zugabe von NaHSO₃ reduziert, bevor der Reaktionsansatz aufkonzentriert wird, der dann in einem organischen Lösungsmittel, wie einem Gemisch aus Chloroform/Methanol aufgenommen und gegen Wasser ausgeschüttelt wird. Die organische Phase wird aufkonzentriert und chromatografiert, in dem man z.B. mit Chloroform verdünnt und dann an einer Kieselgelsäule mit einem Chloroform/Methanol-Gradienten fraktioniert, dessen Methanolanteil steigend ist.

Zum vollständigen Austausch von gegebenenfalls vorhandenen 5'-Hydroxyschutzgruppen (z.B. Monomethoxytritygruppe) gegen Wasserstoff wird das erhaltene Produkt in Methanol/Essigsäure bei Raumtemperatur gerührt und anschließend erneut aufkonzentriert. Der Rückstand wird mit Ether versetzt und in einen Niederschlag überführt, abzentrifugiert und getrocknet und erneut in obiger Weise chromatographiert. Das Produkt wird getrocknet und zum Austausch von gegebenenfalls vorhandenen Silylgruppen gegen Wasserstoff in trockenem organischen Lösungsmittel, wie Tetrahydrofuran gelöst, mit Tetrabutylammoniumfluorid-Trihydrat z.B. in Tetrahydrofuran versetzt, bei Raumtemperatur gerührt und dann aufkonzentriert.

Zum Austausch von gegebenenfalls vorhandenen Aminoschutzgruppen (wie Benzoylgruppen) gegen Wasserstoff wird das erhaltene Produkt mit konzentrierter Ammoniaklösung versetzt und bei Raumtemperatur gerührt. Der Ansatz wird dann aufkonzentriert und das Produkt isoliert, in Wasser gelöst durch Reverse Phase Chromatographie (z.B. RP-18 Säule) z.B. mit einem Wasser/Methanol-Gradienten fraktioniert, dessen Methanolanteil steigend ist. Die produktenthaltenen Fraktionen werden vereinigt, mit einem Kationenaustauscher (H⁺-Form) z.B. auf pH ca. 5.8 eingestellt und vom Austauscher wieder abgetrennt. Das Filtrat wird mit Ammoniak neutralisiert, anschließend konzentriert und dann lyophilisiert, wobei man den gewünschten Duplexwirkstoff erhält.

### d) Erfindungsgemäße pharmazeutische Formulierungen und Anwendungen

Die Kupplung des ethinylierten Nucleosids, wie des therapeutisch hochwirksamen Ethinylcytidins, an ein zweites, ebenfalls wirksames Nucleosidanalogon, resultiert in einem sogenannten Duplex-Wirkstoff, der additive und/oder synergistische Wirkmechanismen auslöst. Dieser Effekt ist dann besonders stark ausgeprägt, wenn beide Monomere unterschiedliche Targets angreifen. Hierdurch kann die applizierte therapeutische Menge der hochpotenten Duplexwirkstoffe im Vergleich zu der für die jeweiligen Monomere so dosiert werden, dass die gewünschte therapeutische Wirkung optimiert, wobei gleichzeitig die unerwünschten toxischen Nebenwirkungen maßgeblich reduziert werden.

Die unterschiedliche Sequenz, eine natürliche 3'-5' oder unnatürliche 5'-5' Phosphodiesterbrücke, in der beide Nucleosidanaloga zum Heterodinucleosid- phosphatanaloga gekuppelt werden können, führt bei der enzymatischen Metabolisierung in vivo zu sehr unterschiedlich, vorausbestimmbaren derivatisierten Metaboliten. Hierdurch kann das therapeutische Spektrum quasi programmiert und maßgebend erweitert werden. Eine Folge davon ist, dass Duplexwirkstoffe bei Resistenzen wirksam sind, gegen die sich die jeweiligen monomeren Nucleosidanaloga als unwirksam erweisen.

Die Überführung von ethinylierten Nucleosiden, wie Ethinylcytidin, in Duplex-Wirkstoffe bedingt auch ein stark verändertes pharmakokinetisches Verhalten, was wiederum zur Optimierung der Therapie beiträgt. Durch die große Variabilität der Derivatisierung sind ethinylierte Duplex-Wirkstoffe je nach der Art der eingeführten Substituenten mit sehr unterschiedlichen Lösungseigenschaften darstellbar. Hierdurch eröffnen sich zahlreiche Möglichkeiten der pharmazeutischen Formulierung, die für das monomere Ethinylcytidin, aufgrund dessen Hydrophilie nicht anwendbar sind.

Ein weiterer Vorteil der erfindungsgemäßen Duplex-Wirkstoffe liegt darin, dass sie zusammen mit einem oder mehreren anderen Wirkstoffen in unterschiedlichen Mengen in Liposomen oder Nanopartikeln eingebaut werden können, wobei synergistische Wirkungen resultieren.

Gegenstand der Erfindung sind damit auch pharmazeutische Mittel, enthaltend mindestens eine Verbindung nach obiger Definition in einem pharmazeutisch verträglichen Träger oder Verdünnungsmittel, wie insbesondere enthalten in Liposomen oder Nanopartikeln.

Weiterhin können erfindungsgemäße Mittel zusätzlich wenigstens einen weiteren pharmakologischen Wirkstoff enthalten, der zur Behandlung von Infektionskrankheiten und/oder Krebserkrankungen geeignet ist.

Als nichtlimitierende Beispiele für weitere Wirkstoffe zur Tumorbehandlung sind zu nennen:
(A) Antineoplastischen Mitteln, wie
   (1) pflanzliche Zytostatika, wie z.B Mistelpräparate,
   (2) chemisch definierte Zytostatika, wie
      a) Alkaloide und Podophyllotoxine, wie z.B. Vinblastin, Vincristin und andere Vinca-Alkaloide und Analoga; Podophyllotoxinderivate, wie Etoposid;
      b) Alkylanzien, wie Nitrosoharnstoffe und Stickstofflost-Analoga, wie z. B. Cyclophosphamid und Estramustin;
      c) zytotoxische Antibiotika, wie Anthracycline und verwandte Substanzen, wie z.B. Daunorubicin, Doxorubicin; Bleomycin und Mitomycin;
      d) Antimetabolite, wie Folsäure-Analoga, wie z.B. Methotrexat, Purin-Analoga, Pyrimidin-Analoga, wie z.B. Cytarabin und Fluoruracil;
   (3) Platinverbindungen, wie Carboplatin, Cisplatin;
   (4) Enzyme und monoklonale Antikörper;
   (5) Endokrin wirkende antineoplastische Mittel, wie
      a) Hormone und verwandte Stoffe, wie z.B. Östrogene, Gestagene, wie z.B. Medroxyprogesteronacetat; Hypothalamushormone, wie Gonadorelin-Analoga, wie z.B. Buserelin;
      b) Hormonantagnisten, wie das Antiestrogen Tamoxifen und andere Antiestrogene; oder das Antiandrogen Flutamid und andere Antiandrogene;
      c) Enzyminhibitoren
(B) Protektiva/Antidota für die antineoplatische Therapie, wie z. B. Folinsäure.

Als nichtlimitierende Beispiele für weitere Wirkstoffe zur Behandlung von Infektionskrankheiten, wie insbesondere AIDS, sind zu nennen: Azidothymidin, Dideoxycytidin, Sanilvudin, Stavudin(1-(2,3-Didesoxy-beta-D-glycero-pent-2-enofuranosyl)-5-methyl-2,4(1H,3H)-pyrimidindion), Dideoxyinosin, rekombinantes (humanes) Interleukin-2, Saquinavir-Mesilat, Interferon alpha, Nevirapin, Abacavir-Sulfat, CD4-Immunoadhesin, Lamivudin, Kynostatin-272, Emtricitabin, Delavirdin-Mesilat, HIV-1-Immunogen, Indinavir-Sulfat, Azidothymidinphosphonat, Calanoid A, Amprenavir, Efavirenz, Ritonavir, Nelfinavir-Mesilat, Gadoliniumtexaphyrin, Enfuvirtid, Buffy coat Interleukin, Semapimodhydrochlorid, Elvucitabin, Canovirin N, Tipranavir, Azodicarbonamid, Tenofovir-disoproxil-fumarat, Atazanavir-Sulfat, Lamivudin/Zidovudin, Sampidin, Dapivirin, Etravirin, Lopinavir/Ritonavir, Adargileukin-alfa, Glyminox, Ancriviroc, O-(2-Hydroxypropyl)-beta-cyclodextrin, Darunavir, Maraviroc, Abacavirsulfat/Lamivudin, sulfoniertes Hesperidin, Rilpivirin, Tenofovir,

Gegenstand der Erfindung ist insbesondere auch Verwendung wenigstens einer Verbindung nach obiger Definition zur Herstellung eines pharmazeutischen Mittels zur Prävention und/oder Therapie von Infektionskrankheiten und/oder Krebserkrankungen.

Die erfindungsgemäßen Verbindungen werden im allgemeinen in Form pharmazeutischer Mittel zur Behandlung eines Individuums, vorzugsweise eines Säugers, insbesondere eines Menschen, eingesetzt. So werden die Verbindungen gewöhnlich in Form von pharmazeutischen Zusammensetzungen verabreicht, die einen pharmazeutisch verträglichen Exzipienten mit wenigstens einem erfindungsgemäßen ethinylierten Nucleosidphosphatanalogon, gegebenenfalls auch ein Gemisch mehrerer erfindungsgemäßer Verbindungen, und gegebenenfalls weitere für den jeweiligen gewünschten therapeutischen Effekt brauchbare Wirkstoffe umfassen. Diese Zusammensetzungen können beispielsweise auf oralem, rektalem, transdermalem, subkutanem, intravenösem, intramuskulärem oder intranasalem Weg verabreicht werden.

Beispiele geeigneter pharmazeutischer Formulierungen sind feste Arzneiformen, wie Pulver, Puder, Granulate, Tabletten, Pastillen, Sachets, Cachets, Dragees, Kapseln wie Hart- und Weichgelatinekapseln, Suppositorien oder vaginale Arzneiformen; halbfeste Arzneiformen, wie Salben, Cremes, Hydrogele, Pasten oder Pflaster, sowie flüssige Arzneiformen, wie Lösungen, Emulsionen, insbesondere Öl-in-Wasser-Emulsionen, Suspensionen, beispielsweise Lotionen, Injektions- und Infusionszubereitungen, Augen- und Ohrentropfen. Auch implantierte Abgabevorrichtungen können zur Verabreichung erfindungsgemäßer Verbindungen verwendet werden. Ferner können auch Liposomen, Mikrosphären oder Polymermatrizes zur Anwendung kommen.

Bei der Herstellung der Mittel werden erfindungsgemäße Verbindungen gewöhnlich mit einem Exzipienten vermischt oder verdünnt. Exzipienten können feste, halbfeste oder flüssige Materialien sein, die als Vehikel, Träger oder Medium für den Wirkstoff dienen.

Zu geeigneten Exzipienten gehören beispielsweise Lactose, Dextrose, Succrose, Sorbitol, Manitol, Stärken, Akaziengummi, Calciumphosphat, Alginate, Traganth, Gelantine, Calciumsilikat, mikrokristalline Cellulose, Polyvinylpyrrolidon, Cellulose, Wasser, Sirup und Methylcellulose. Ferner können die Formulierungen pharmazeutisch akzeptable Träger oder übliche Hilfsstoffe, wie Gleitmittel, beispielsweise Talg, Magnesiumstearat und Mineralöl; Netzmittel; emulgierende und suspendierende Mittel; konservierende Mittel, wie Methyl- und Propylhydroxybenzoate; Antioxidantien; Antireizstoffe; Chelatbildner; Dragierhilfsmittel, Emulsionsstabilisatoren Filmbildner; Gelbildner; Geruchsmaskierungsmittel; Geschmackskorrigentien; Harze; Hydrokolloide; Lösemittel; Lösungsvermittler; Neutralisierungsmittel; Permeationsbeschleuniger; Pigmente; quaternäre Ammoniumverbindungen; Rückfettungs- und Überfettungsmittel; Salben-, Creme- oder Öl-Grundstoffe; Silikon-Derivate; Spreithilfsmittel; Stabilisatoren; Sterilanzien; Suppositoriengrundlagen; Tabletten-Hilfsstoffe, wie Bindemittel, Füllstoffe, Gleitmittel, Sprengmittel oder Überzüge; Treibmittel; Trocknungsmittel; Trübungsmittel; Verdickungsmittel; Wachse; Weichmacher; Weißöle umfassen. Eine diesbezügliche Ausgestaltung beruht auf fachmännischem Wissen, wie beispielsweise in Fiedler, H.P., Lexikon der Hilfsstoffe für Pharmazie, Kosmetik und angrenzende Gebiete, 4. Auflage, Aulendorf: ECV-Editio-Kantor-Verlag, 1996, dargestellt ist.

Bevorzugte übliche Träger sind beispielsweise Mannit, Glucose, Dextrose, Albumine oder ähnliches; bevorzugte Verdünnungsmittel sind im wesentlichen physiologische Kochsalzlösungen oder eine 5%ige Glucoselösung. Weiterhin ist es üblich, derartige Lösungen mit geeigneten Reagenzien, beispielsweise Phosphaten, abzupuffern.

Für eine bessere Applikation der erfindungsgemäßen Verbindungen können Zusammensetzungen bereitgestellt werden, welche die erfindungsgemäßen Verbindungen in Kombination mit einem organischen Träger enthalten. Darüber hinaus können alle weiteren, für die Zubereitung von pharmazeutischen Mitteln üblichen Excipienten hinzugesetzt werden, vorausgesetzt, sie der bestimmungsgemäße Gebrauch derartiger Zusammensetzung aus dem organischen Träger und den erfindungsgemäßen Verbindungen wird nicht beeinträchtigt.

Eine bevorzugte Ausführungsform solcher Zusammensetzungen sieht die Assoziation der erfindungsgemäßen Verbindungen in Form von uni- bis oligolamellaren Liposomen mit einem Durchmesser von maximal 0.4 µm vor. Zur Liposomenbildung können alle an sich bekannten Verfahren der Liposomendarstellung verwendet werden, wie beispielsweise Ultraschall, Gelchromatographie, Detergenzanalyse, Hochdruckfiltration. Die jeweils eingeführten lipophilen Reste beeinflussen maßgeblich die Größe und Stabilität der Liposomen, die sich aus den jeweiligen Glycerylnucleotiden zusammen mit weiteren Lipidkomponenten bilden (vgl. auch Liposomes: From Physical Structure to Therapeutic Applications in: Research monographs in cell and tissue physiology Bd.7, G.G. Knight Hrsg., Elsevier (1981).

Eine weitere bevorzugte Möglichkeit, die erfindungsgemäßen Verbindungen mit einem organischen Träger zu verbinden, ist der Einschluss der Verbindungen in biologisch verträgliche Nanopartikel. Als Nanopartikel werden organisch-chemische Polymere bezeichnet, denen bei der Polymerisation die erfindungsgemäßen Verbindungen zugesetzt werden, so dass sie mit einer gewissen Effizienz in das Nanopartikel eingeschlossen werden (vgl. Bender et al., Antimicrobial agents and Chemotherapy (1996), 40 (6) 1467-1471).

In einer bevorzugten Ausführungsform kann die erfindungsgemäße Zusammensetzung oder der erfindungsgemäße Wirkstoff weitere Komponenten umfassen oder damit kombiniert werden, die eine spezifische Anreicherung im Bereich der zu therapierenden Zellen und/oder Organen begünstigen. Dabei kann beispielsweise die Zusammensetzung der Liposomen so gewählt werden, dass die Liposomen zusätzlich mit Molekülen, wie z.B. Antikörpern, geladenen Lipiden, oder mit hydrophilen Kopfgruppen modifizierten Lipiden versehen werden, damit die Zusammensetzung sich bevorzugt in den zu therapierenden Zellen und/oder Organen oder in deren Umgebung anreichert. Eine solche Zusammensetzung mit spezifisch gegen Tumorzellen, virusinfizierten Zellen und/oder Organe gerichteten Molekülen erhöht die therapeutische Wirkung der Arzneimittel und reduziert gleichzeitig die Toxizität für nicht infizierte Gewebe.

Derartige Zusammensetzungen können zu einem pharmazeutischen Mittel verarbeitet werden, das neben den erfindungsgemäßen Verbindungen und gegebenenfalls dem organischen Träger weiterhin übliche Träger- und/oder Verdünnungsmittel und/oder Hilfsstoffe enthält. Übliche Trägermittel sind beispielsweise Mannit, Glucose, Dextrose, Albumine oder ähnliches, während als Verdünnungsmittel im wesentlichen physiologische Kochsalzlösungen oder eine 5%ige Glucoselösung dient. Weiterhin ist es üblich, die Lösungen mit geeigneten Reagenzien, beispielsweise Phosphaten, abzupuffern. Darüber hinaus können alle weiteren, für die Zubereitung von pharmazeutischen Mitteln üblichen Zusatzstoffe hinzugesetzt werden, vorausgesetzt, die Zusammensetzung aus dem organischen Träger und den erfindungsgemäßen Verbindungen wird nicht negativ beeinflusst.

Durch die Überführung in Duplex-Wirkstoffe lassen sich aber nicht nur die enzymatische Hydrolysebeständigkeit erhöhen und die Applikationsformen deutlich erweitern, sondern überraschenderweise auch zytostatische und virusstatische Wirkungen optimieren.

Die Duplex-Wirkstoffe lassen sich gegen maligne Erkrankungen der blutbildenden Zellen und solide Tumore einsetzen. Durch die verbesserte zytostatische Wirkung treten sehr viel weniger gravierende Nebenwirkungen auf. Es können höhere Dosen der zytostatisch wirksamen erfindungsgemäßen Verbindungen eingesetzt werden und es kann in zeitlichen Intervallen therapiert werden.

Insbesondere sind die erfindungsgemäßen Verbindungen einsetzbar zur Prophylaxe und/oder Therapie folgender Tumorerkrankungen: Leukämie, Lungenkrebs, Darmkrebs, Krebs des Zentralnervensystems, Melanomen, Ovarialkrebs, Nierenkrebs, Prostatakrebs und Brustkrebs.

Überraschenderweise zeigen die erfindungsgemäßen Duplex-Wirkstoffe auch virusstatische Wirkungen, so dass sie in der Chemotherapie von virusbedingten Infektionen und zur Überwindung von Arzneimittelresistenzen verwendbar sind.

Insbesondere sind die erfindungsgemäßen Verbindungen einsetzbar zur prophylaxe und/oder Therapie folgender Viruserkrankungen: AIDS (HIV-Infektion), Hepatitis A, B und C, Herpes und CMV-Infektionen.

Die Erfindung wird nunmehr anhand der folgenden nichtlimitierenden Beispiele näher beschrieben. Soweit keine anderen Angaben gemacht werden, erfolgt die Herstellung, Formulierung und Testung erfindungsgemäßer Wirkstoffe und Zusammensetzungen unter Anwendung geängiger Methoden des Standes der Technik.

### Experimenteller Teil

### Beispiel 1

### Darstellung von 5-Fluoro-2'-desoxyuridylyl-(3'-5')-3'-C-ethinylcytidin

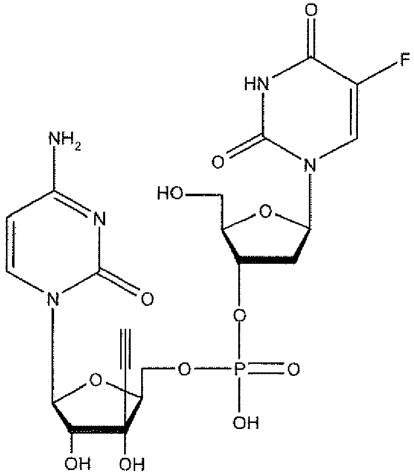

### a) Herstellung Edukte

### Edukt 1:

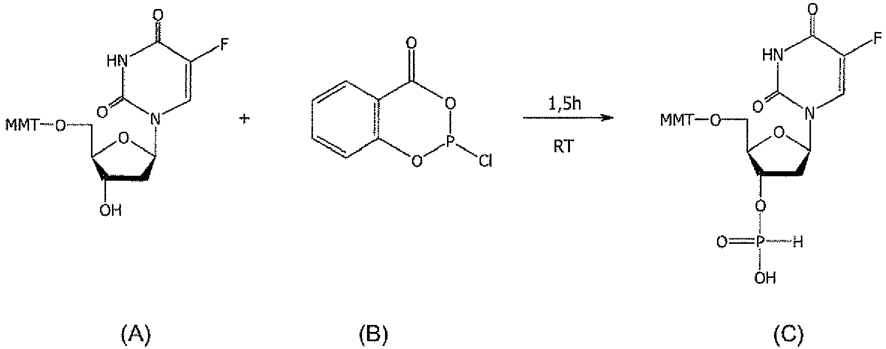

5'-O-(4-Monomethoxytrityl)-5-fluoro-2'-desoxyuridin-3'-hydrogenphosphonat (C) wird in einem zweistufigen Prozess hergestellt. Zunächst wird 5'-O-(4-Monomethoxytrityl)-5-fluoro-2'-desoxyuridin (A) wie in Ludwig, P.S. et al., European Journal of Medical Chemistry 2005 494-504 (vgl. dortige Verbindung (1)) hergestellt. Anschließend wird die 3'-Hydrogenphosphatgruppe auf folgendem Weg eingeführt. Hierzu wird eine Lösung von 20 g (39 mmol) 5'-0-(4-Monomethoxytrityl)-5-fluoro-2'-desoxyuridin (A) in 50 ml wasserfreiem Pyridin mit 90 ml wasserfreiem Dioxan verdünnt und anschließend mit 50 ml Dioxan versetzt, in dem 11 g (54 mmol) Salicylchlorophosphit (B) gelöst sind. Der Reaktionsansatz wird 1.5 h bei Raumtemperatur gerührt. Der hierbei ausfallende Niederschlag wird anschließend abgesaugt und mit kaltem Ether nachgewaschen. Filtrat und Waschflüssigkeit werden vereinigt, mit 50 ml gesättigter Natriumcarbonatlösung versetzt und im Rotationsverdampfer zum Schaum konzentriert, der dann in ca. 300 ml eines Gemisches aus Chloroform/Methanol (95:5) gelöst, an einer Kielgelsäule mit einem Chloroform/Methanol-Gradienten fraktioniert wird, dessen Methanolanteil steigend ist. Die vereinigten Produkt enthaltenden Fraktionen werden im Rotationsverdampfer zum Schaum konzentriert und ergeben nach dem Trocknen im Vakuum 20,5 g (35 mmol) 5'-0-(4-Monomethoxytrityl)-5-fluoro-2'-desoxyuridin-3'-hydrogenphosphonat (C).

### Edukt 2:

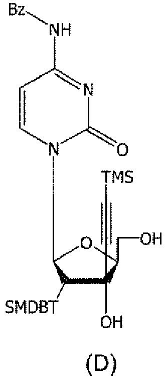

N⁴-Benzoyl-2'-O-(tert.-butyldimethylsilyl)-3'-C-(trimethylsilylethinyl)cytidin (D) wird nach der Synthesevorschrift in Ludwig, P.S. et al., Synthesis 2002 , 16 2387-2392 (vgl. dortige Verbindung (6)) hergestellt.

### b) 3'-5'-Verknüpfung der Edukte

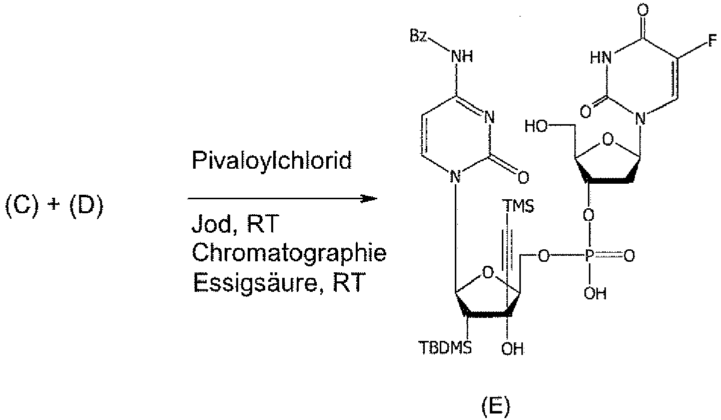

19.2 g (33 mol) 5'-O-(4-Monomethoxytrityl)-5-fluoro-2'-desoxyuridin-3'-hydrogenphosphonat (C) werden zusammen mit 18.4 g (33 mmol) N⁴-Benzoyl-2'-O-(tert.-butyldimethylsilyl)-3'-C-(trimethylsilylethinyl)cytidin (D) in 100 ml wasserfreiem Pyridin gelöst. Die auf ca. 10°C abgekühlte Lösung wird unter Feuchtigkeitsausschluss mit 24 ml (195 mmol) Pivaloylchlorid versetzt, 5 min. bei Raumtemperatur kräftig ge-schüttelt, dann schnell auf ca. 0°C gekühlt und mit 20 ml Wasser versetzt. Der Reaktionsansatz wird bei Raumtemperatur einige Minuten gerührt, dann mit 160 ml einer Lösung von 25.4 g Iod in 450 ml Tetrahydrofuran versetzt und anschließend 1 h bei Raumtemperatur gerührt. Überschüssiges Iod wird durch Zugabe von festem NaHSO₃ reduziert, bevor der Reaktionsansatz im Rotationsverdampfer zum Sirup konzentriert wird, der dann in 300 ml eines Gemisches aus Chloroform/Methanol (9:1) aufgenommen und gegen ca. 250 ml Wasser ausgeschüttelt wird. Die organische Phase wird am Rotationsverdampfer zum Sirup konzentriert, der mit 300 ml Chloroform verdünnt und dann an einer Kieselgelsäule mit einem Chloroform/Methanol-Gradienten fraktioniert wird, dessen Methanolanteil steigend ist. Im Verlauf der Chromatographie wird bereits die Monomethoxytritylgruppe bei einem Teil des Produkts gegen Wasserstoff getauscht. Die Fraktionen, die das Produkt mit und ohne Monomethoxytritylrest enthalten ergeben nach der Konzentration am Rotationsverdampfer ca. 32 g Schaum. Zum vollständigen Austausch der Monomethoxytritygruppe gegen Wasserstoff wird der erhaltene Schaum zusammen mit 50 ml Methanol in 60 ml 80%iger Essigsäure 24h bei Raumtemperatur gerührt und anschließend am Rotationsverdampfer wieder zum Schaum konzentriert. Der Schaum wird mit ca. 120 ml Ether versetzt, kräftig geschüttelt und hierbei in einen Niederschlag überführt, der nach dem Abzentrifugieren und Trocknen ca. 25 g Feststoff ergibt. Der Feststoff wird in ca. 250 ml Chloroform gelöst und an einer Kieselgelsäule mit einem Chloroform/Methanol-Gradienten fraktioniert, dessen Methanolanteil steigend ist. Produktenthaltene Fraktionen werden am Rotationsverdampfer zum Schaum konzentriert, der bei Etherzugabe in einen Feststoff von (E) übergeht, der nach dem Trocknen 19 g ergibt.

### c) Herstellung des Endprodukts

Der gemäß Stufe b) erhaltene Feststoff (E) wird zum Austausch der Silylgruppen gegen Wasserstoff in 170 ml trockenem Tetrahydrofuran gelöst, mit 85 ml einer 1M Lösung von Tetrabutylammoniumfluorid-Trihydrat in Tetrahydrofuran versetzt, verschlossen 3 Tage bei Raumtemperatur gerührt und dann am Rotationsverdampfer zum Sirup konzentriert, wobei man Verbindung (F) erhält

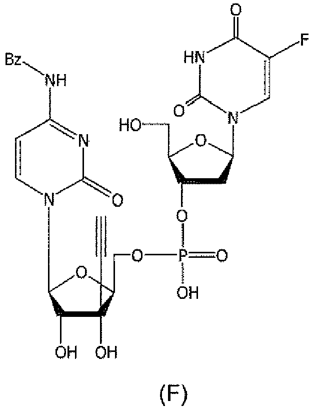

Zum anschließenden Austausch des Benzoylrestes gegen Wasserstoff wird der erhaltene Sirup mit ca. 300 ml einer 33%igen Ammoniaklösung versetzt und verschlossen 5 Tage bei Raumtemperatur gerührt. Der Ansatz wird dann am Rotationsverdampfer auf ca. 250 ml konzentriert. Der ausgefallene feine Niederschlag wird abzentrifugiert. Der Überstand wird lyophilisiert. Das Lyophilisat wird in ca. 60 ml Wasser gelöst und an einer präparativen RP-18 Säule mit einem Wasser/Methanol-Gradienten fraktioniert, dessen Methanolanteil steigend ist. Die produktenthaltenen Fraktionen, die bei einem Methanolanteil des Gradienten von ca. 15%-40% die Säule verlassen, werden vereinigt, mit einem Kationenaustauscher (H⁺-Form) auf pH ca. 5.8 eingestellt und vom Austauscher abgetrennt. Das Filtrat wird mit Ammoniak neutralisiert, anschließend konzentriert und dann lyophilisiert. Hierbei werden 10,6 g des gewünschten Produkts als Tetrabutylammoniumsalz erhalten. Die berechneten Molekülmassen für die anionische Form 574.40 und die Tetrabutylammoniumsalzform 815.8 werden im FAB-Massenspektrum durch die Molekülpeaks 574.0 und 815.8 [M-H]⁻ bestätigt.

### Beispiel 2 (Referenzbeispiel)

### Darstellung von 5-Fluoro-2'-desoxyuridylyl-(5'-5')-3'-C-ethinylcytidin

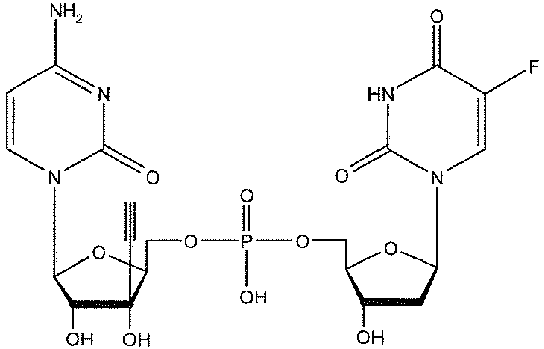

### a) Herstellung der Edukte:

### Edukt 1:

3'-4-Di-O-benzoyl-5-fluaro-2'-desoxyuridin-5'-hydrogenphosphanat wird in einem zweistufigen Prozess aus 5'-0-(4-Monomethoxytrityl)-5-fluoro-2'-desoxyuridin hergestellt.

Hierzu werden 26 g (50 mmol) 5'-0-(4-Monomethoxytrityl-5-fluoro-2'-desoxyuridin in 150 ml wasserfreiem Pyridin gelöst. Anschließend wird die gekühlte Lösung mit 56 g (400 mmol) Benzoylchlorid versetzt und 8 h unter Feuchtigkeitsausschluß bei Raumtemperatur gerührt. Die Reaktion wird beendet, indem zu der gekühlten Lösung 130 ml einer gesättigten Natriumcarbonatlösung gegeben werden. Der Reaktionsansatz wird dann im Rotationsverdampfer zum Sirup konzentriert, der mit 300 ml Chloroform verdünnt und anschließend mit 130 ml einer gesättigten Natriumcarbonatlösung ausgeschüttelt wird. Die abgetrennte Chloroform-Phase wird zum Sirup konzentriert, der dann mit 200 ml eines Gemisches aus Chloroform/Petrolether (1:1) verdünnt und an einer Kieselgelsäule chromatographiert wird. Hierbei wird die Säule mit einem Chloroform/Petrolethergradienten mit steigendem Chloroformanteil eluiert. Die vereinigten, Produkt enthaltenden Fraktionen ergeben nach dem Konzentrieren im Vakuum ca. 46 g eines Schaumes.

Zum Austausch der 5'-0-(4-Monomethoxytrityl)-Gruppe gegen Wasserstoff wird der Schaum im 100 ml Aceton aufgenommen, in dem 16 g Toluolsulfonsäuremonohydrat gelöst sind. Nach 20 min Rühren bei Raumtemperatur wird der Reaktionsansatz mit 50 ml einer gesättigten Natriumcarbonatlösung versetzt, im Vakuum zum Sirup konzentriert, der dann mit 500 ml Chloroform und 100 ml Wasser verdünnt wird. Die abgetrennte Chloroformphase wird an einer Kieselgelsäule fraktioniert, wobei zur Elution zunächst ein Chloroform/Petrolethergradient mit steigendem Chloroformanteil und anschließend Ether verwendet werden. Die vereinigten Produkt enthaltenden Fraktionen werden im Rotationsverdampfer zum Schaum konzentriert, der nach dem Trocknen im Vakuum 21 g (46 mmol) 3'-4-Di-O-benzoyl-5-fluoro-2'-desoxyuridin ergibt.

Zur Einführung der 5'-Hydrogenphosphonatgruppe wird der Schaum in 90 ml wasserfreiem Pyridin gelöst. Die Lösung wird mit 180 ml wasserfreiem Dioxan verdünnt, dann mit weiteren 75 ml Dioxan versetzt, in denen 13 g (64 mmol) Salicylchlorophosphit gelöst sind und anschließend 2 h bei Raumtemperatur gerührt. Der Reaktionsansatz wird mit 12 ml einer gesättigten Natriumhydrogencarbonatlösung versetzt, dann im Vakuum zum Sirup konzentriert, der in 500 ml Chloroform aufgenommen und dreimal mit jeweils 200 ml einer Mischung aus Wasser/gesättigter Natriumchloridlösung/Methanol (1:1:2) ausgeschüttelt wird. Die Chloroform-Phase wird zum Sirup konzentriert, der mit 150 ml Chloroform verdünnt und unter Rühren in 1.5 l Ether eingetragen wird. Der hierbei ausfallende Niederschlag wird abgesaugt, getrocknet und anschließend mit Ether ca. 70 h extrahiert, wobei 20 g (39 mmol) 3'-4-Di-0-benzoyl-5-fluoro-2'-desoxyuridin-5'-hydrogenphosphonat verbleiben.

### Edukt 2:

N⁴-Benzoyl-2'-O-(tert.-butyldimethylsilyl)-3'-C-(trimethylsilylethinyl)cytidin wird nach der Synthesevorschrift in Ludwig, P.S. et al., Synthesis 2002 , 16 2387-2392 (vgl. dortige Verbindung (**6**)) hergestellt.

### b) 5'-5'-Verknüpfung der Edukte

11.0 g (21.2 mmol) 3'-4- Di-O-benzoyl-5-fluoro-2'-desoxyuridin-5'-hydrogenphosphonat werden zusammen mit 9.5 g (17 mmol) N⁴-Benzoyl-2'-O-(tert.-butyldimethylsilyl)-3'-C-(trimethylsilylethinyl)cytidin in ca. 100 ml wasserfreiem Pyridin gelöst. In Analogie zu Stufe b) aus Beispiel 1 wird durch Zusatz von 13 ml (10,6 mmol) Pivaloylchlorid die Kondensation gestartet, die Reaktion nach 5 min. durch Zugabe von 10 ml Wasser abgebrochen und das Kondensat anschließend mit 82 ml einer Lösung von 25,4 g Iod in 450 ml Tetrahydrofuran oxidiert. Nach der Aufarbeitung des Reaktionsansatzes, der chromatographischen Reinigung an einer Kieselgelsäule und der anschließenden Etherfällung werden 13 g eines Feststoffes erhalten

### c) Herstellung des Endprodukts

In dem gemäß b) erhaltenen Feststoff werden in Analogie zu Stufe c) aus Beispiel 1 zunächst die Silylgruppen gegen Wasserstoff getauscht, indem der Feststoff mit 45 ml Tetrahydrofuran und 22 ml eine 1 M Lösung von Tetrabutylammoniumfluorid-Trihydrat in Tetrahydrofuran 3 Tage bei Raumtemperatur behandelt und dann zum Sirup konzentriert wird. Anschließend wird der Sirup zum Austausch der Benzoylreste gegen Wasserstoff in 80 ml eine 33%igen Ammoniaklösung 5 Tage verschlossen gerührt. Der aufgearbeitete Reaktionsansatz wird lyophilisiert und das erhaltene Lyophilisat wie unter Stufe c) im Beispiel 1 beschrieben an einer präparativen RP-18 Säule fraktioniert. Die Produktfraktionen werden in 5,8 g eines Lyophilisats überführt. Die berechneten Molekülmassen für die anionische Form 574.40 und die Tetrabutylammoniumsalzform 815.8 werden im FAB-Massenspektrum durch die Molekülpeaks 574.0 und 815.8 [M-H]⁻ bestätigt.

### Testbeispiel 1

### Bestimmung der zytostatische Wirkung in vitro

Die in vitro zytostatische Wirkung der erfindungsgemäßen Verbindungen lässt sich durch folgende Versuchsanordnung zeigen.

Als Testsystem dienen Tumorzellinien, deren 100%ige Wachstumshemmung (TGI) durch die erfindungsgemäßen Verbindungen bei verschiedenen Konzentrationen ermittelt wird. Ferner wird die Toxizität (LC₅₀) der Verbindung auf diese Zellen ermittelt. Eine Serie von Mikrotiterplatten wird am Tag 0 mit den Tumorzellen beimpft und für 24 h vorinkubiert. Danach wird die erfindungsgemäße Verbindung den Zellen in fünf jeweils 10-fach verdünnter Konzentration zugegeben, ausgehend von der höchsten löslichen Konzentration. Es folgt eine 48-stündige Inkubation, an deren Ende die Zellen in situ fixiert, gewaschen und getrocknet werden. Anschließend wird Sulforhodamin B(SRB), ein pinker Farbstoff der an die fixierten Zellen gebunden wird, zugefügt und die Zellen erneut gewaschen. Der verbleibende Farbstoff spiegelt die adhärente Zellmasse wieder und wird spektroskopisch bestimmt. Die automatisch erfassten Daten werden per Computer ausgewertet und führen für die erfindungsgemäße Verbindung 5-Fluoro-2'-deoxyuridylyl-(5'-5')-3'-C-ethinylcytidin und 5-Fluoro-2'-deoxyuridylyl-(3'-5')-3'-C-ethinylcytidin zu folgenden Ergebnissen, wobei die Daten für das 3'-5'-verknüpfte Dimer im Fettdruck oberhalb der Daten für das 5'-5'- verknüpfte Isomer aufgeführt sind

| **In vitro Testergebnisse von** | | | |
|---|---|---|---|
| **Tumorzelllinie** | | **TGI in mol/l** | **LC₅₀ in mol/l** |
| **Lunge** | | | |
| **A549/ATCC** | | **2,96 E-5** | **> 1,00 E-4** |
| | | > 1,00E-4 | > 1,00 E-4 |
| | | | |
| **EKVX** | | **6,37 E-6** | **> 1,00 E-4** |
| | | > 1,00 E-4 | > 1,00 E-4 |
| | | | |
| **HOP-92** | | **2,18 E-6** | **> 1,00 E-4** |
| | | 2,15 E-5 | > 1,00 E-4 |
| | | | |
| **NCI-H522** | | **1,15 E-6** | > **1,00 E-4** |
| | | > 1,00 E-4 | > 1,00 E-4 |
| | | | |

| **Darm** | | | |
|---|---|---|---|
| **HCC-2998** | | **1,09 E-7** | **1,73 E-6** |
| | | 2,95 E-6 | 5,72 E-5 |
| | | | |
| **HCT-15** | | **1,51 E-5** | **> 1,00 E-4** |
| | | > 1,00 E-4 | > 1,00 E-4 |
| | | | |

| **Zentralnervensystem** | | | |
|---|---|---|---|
| **SF-295** | | **1,07 E-6** | **> 1,00 E-4** |
| | | 2,06 E-5 | > 1,00 E-4 |
| | | | |
| **SF-539** | | **1,67 E-7** | **3,22 E-5** |
| | | 2,01 E-6 | > 1,00 E-4 |
| | | | |
| **SNB-75** | | **8,82 E-7** | **4,72 E-6** |
| | | > 1,00 E-4 | > 1,00 E-4 |
| | | | |

| **Melanom** | | | |
|---|---|---|---|
| **LOX IMVI** | | **3,48 E-7** | **9,15 E-5** |
| | | 1,00 E-4 | 1,00 E-4 |
| | | | |
| **SK-MEL-2** | | **4,24 E-7** | **6,95 E-5** |
| | | 5,78 E-6 | 7,64 E-5 |
| **Ovarien** | | | |

| | | | |
|---|---|---|---|
| **IGROV1** | | **1,29 E-5** | **> 1,00 E-4** |
| | | > 1,00 E-4 | > 1,00 E-4 |
| | | | |

| **Niere** | | | |
|---|---|---|---|
| **ACHN** | | **1,52 E-6** | **> 1,00 E-4** |
| | | > 1,00 E-4 | > 1,00 E-4 |
| | | | |
| **CAK-1** | | **8,29 E-8** | **> 1,00 E-4** |
| | | > 1,00 E-4 | > 1,00 E-4 |

| **Prostata** | | | |
|---|---|---|---|
| **DU-145** | | **4,40 E-7** | **> 1,00 E-4** |
| | | > 1,00 E-4 | > 1,00 E-4 |
| | | | |

| **Brust** | | | |
|---|---|---|---|
| **MCF7** | | **1**,**37 E**-**7** | **> 1,00E-4** |
| | | > 1,00 E-4 | > 1,00 E-4 |
| | | | |
| **BT**-**549** | | **3,46 E-7** | **6,39 E-6** |
| | | 1,16 E-5 | > 1,00 E-4 |
| | | | |
| **T-47D** | | **2,67 E-5** | **> 1,00 E-4** |
| | | > 1,00 E-4 | > 1,00 E-4 |

Es zeigt sich, dass der 3'-5'-verknüpfte Duplexwirkstoffe (Beispiel 1) deutlich wirksamer als der 5'-5'- verknüpfte Duplexwirkstoff (Beispiel 2) ist.

### Testbeispiel 2

### Bestimmung der in vivo-Antitumoraktivität im LOX IMVI Melanom Xenograft-Modell

Der erfindungsgemäße Duplex-Wirkstoff, hergestellt gemäß Beispiel 1 wurde in dem etablierten LOX IMVI Xenograft-Modell für solide Tumoren auf Wirksamkeit getestet.

### a) Versuchsdurchführung:

Eine Zellsuspension von 5 x 10⁶ Tumorzellen (LOX IMVI, Zelllinie 01/A/1) wurde weiblichen athymischen nackten Mäusen (Animal Production Area, Frederick, Maryland) subkutan implantiert. Die intraperitoneale Verabreichung des Wirkstoffs startete 3 Tage nach Tumorimplantation. Dazu wurde der Wirkstoff, gelöst in 10 % DMSO/Kochsalzlösung plus Tween® 80, verabreicht.

Drei Behandlungsgruppen von je acht Tieren erhielten den Wirkstoff in Dosen von 25,0, 16,75 bzw. 11,2 mg/kg je Injektion. Die Kontrollgruppe, umfassend sechzehn Mäuse erhielt entsprechende Volumina Injektionslösung ohne Wirkstoff. Die Verabreichung erfolgte jeweils in einem viertägigen Intervall und umfasste insgesamt 5 Behandlungen. Für die Auswertung der Versuche wurden die T/C-Werte bestimmt.

Für ein Modell basierend auf soliden Tumoren ist es erforderlich, dass ein T/C-Wert von ≤40 erreicht werden muss, um als wirksam angesehen zu werden.

### b) Ergebnis:

Eine Behandlung mit 25 mg/kg/injektion war toxisch. Die Behandlungsgruppe mit 16,75 mg/kg/Injektion zeigte einen maximalen T/C-Wert von 7 am Tag 11 nach Tumorimplantation. Die Behandlungsgruppe mit 11,2 mg/kg/Injektion zeigte einen optimalen T/C-Wert von 16 am Tag 13.

Es zeigte sich somit, dass der getestete 3'-5'-verknüpfte Duplex-Wirkstoff (Beispiel 1) im vorliegenden Tumormodell signifikante *in vivo*-Aktivität besitzt.

### Schlussbemerkung zu dem Testbeispielen 1 und 2:

Die Versuchsergebnisse belegen eindeutig den überraschenden Befund, dass erfindungsgemäße 3'-5'-verknüpfte Duplexwirkstoffe *in vitro* und *in vivo* Antitumoraktivität besitzen. Zudem sind die 3'-5'-verknüpften Duplexwirkstoffe signifikant wirksamer als die entsprechenden 5'-5'-verknüpften Duplexwirkstoffe. Letzterer Befund ist um so überraschender, weil für den Fachmann nicht ohne weiteres zu erwarten war, dass die Einführung einer sterisch möglicherweise hindernden Ethinylgruppe in eine Prodrug, die ein über eine natürliche Phosphodiesterbrücke (d.h. 3'-5'-Phosphodiesterbrücke) verknüpftes Wirkstoffpaar aufweist, keinen inhibierenden Einfluss auf die Spaltung der Phosphodiesterbindung unter Freisetzung der Wirkstoffe zeigt.

Auf die Offenbarung der hierin zitierten Druckschriften wird ausdrücklich Bezug genommen.

## Patentansprüche

1. Ethinylierte Heterodinucleosidphosphatanaloga der Formel I 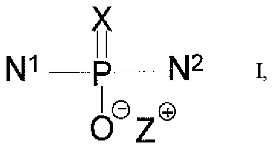 worin
X für O oder S steht;
Z für H oder das korrespondierende Säureadditionssalz dieser Verbindung steht;
N¹ für einen Nukleosidrest der Formel IV 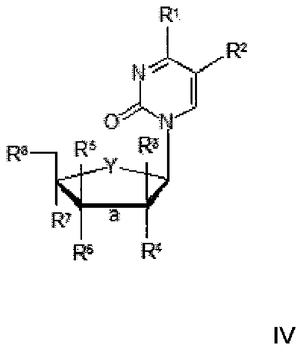 steht, der nicht ethyniliert ist, worin:
R¹ für Hexadecyl-, Palmitoyl-, Oleoylamino oder Hydroxyl steht;
R² für H oder Fluor steht;
R³, R⁴ gleich oder verschieden sind und für H, Hydroxyl, Fluor steht;
R⁵, R⁷ für H steht;
R⁶ für ein Sauerstoffatom steht, mit dem N¹ mit P verbrückt ist,
R⁸ für Hydroxyl, Azido oder H steht und
Y für O oder S steht
und N² für einen Nukleosidrest der Formel IV steht, der ethinyliert ist, worin
R¹ für Amino
R², R³ R⁷ für H;
R⁴, R⁶ für Hydroxyl;
R⁵ für Ethinyl;
R⁸ für ein Sauerstoffatom steht, mit dem N² mit P verbrückt ist und
Y für O oder S steht.

2. Verbindungen nach Anspruch 1, worin N² für einen Nukleosidrest der Formel IV steht der ethinyliert ist, und N¹ für einen Nukleosidrest der Formel IV steht, der nicht ethinyliert ist, worin
R¹ für Hydroxyl steht;
R² für Fluor steht;
R³, R⁴, R⁵ und R⁷ für H stehen,
R⁶ für -O- steht, und
R⁸ für Hydroxyl steht.

3. Verbindung nach einem der vorhergehenden Ansprüche, die 5-Fluoro-2'-desoxyuridylyl-(3'-5')-3'-C-ethinylcytidin ist.

4. Pharmazeutisches Mittel, enthaltend mindestens eine Verbindung nach einem der vorhergehenden Ansprüche in einem pharmazeutisch verträglichen Träger oder Verdünnungsmittel.

5. Mittel nach Anspruch 4, enthalten in Liposomen oder Nanopartikel.

6. Mittel nach Anspruch 4 oder 5, zusätzlich enthaltend wenigstens einen weiteren pharmakologischen Wirkstoff, der zur Behandlung von Infektionskrankheiten und/oder Krebserkrankungen geeignet ist.

7. Verwendung wenigstens einer Verbindung nach einem der Ansprüche 1 bis 3 zur Herstellung eines pharmazeutischen Mittels zur Prävention und/oder Therapie von Infektionskrankheiten und/oder Krebserkrankungen.

8. Verfahren zur Herstellung von ethinylierten Heterodinucleosidphosphatanaloga nach einem der Ansprüche 1 bis 3, wobei man zwei Nukleoside der allgemeinen Formeln Va und Vb
L¹-N¹ (Va)
L² -N² (Vb)
worin
N¹ und N² wie oben definiert sind und gegebenenfalls Schutzgruppen aufweisen; wobei wenigstens eine der Gruppen N¹ und N² am glycosidischen Rest eine Ethinyl- oder geschützte Ethinyl-Gruppe trägt; und
L¹ und L² am glycosidischen Rest von N¹ bzw. N² gebundene, miteinander reaktive Gruppen darstellen, wobei eine der Gruppen L¹ und L² für eine Hydroxy- oder Mercaptogruppe und der andere für eine Hydrogenphosphonat- oder Thiohydrogenphosphonatgruppe steht, und wobei eine der Gruppen L¹ und L² ringständig und die andere endständig gebunden ist;
in Gegenwart eines Säurechlorids kondensiert und das Kondensationsprodukt anschließend oxidiert, und gegebenenfalls vorhandenen Schutzgruppen entfernt.

9. Verfahren nach Anspruch 8, wobei man jeweils zwei Nukleoside der Formeln Va und Vb umsetzt, wobei die Nukleoside Va und Vb einer Verbindung der obigen allgemeinen Formel IV entsprechen, worin
X und a die oben angegebenen Bedeutungen besitzen,
L¹ und L² an Stelle eines der Reste R⁶ oder R⁸ enthalten sind,
die Reste R¹ bis R⁸ im Übrigen die in den Ansprüchen 1 bis 7 angegebene Bedeutung besitzen;
die Reste R¹ und R³ bis R⁸ zusätzlich auch für eine acylierte Hydroxygruppe, deren Acylrest linear oder verzweigt ist, 1-24 C-Atome und 1 oder 2 Doppelbindungen aufweist und durch einen aromatischen Rest substituiert sein kann, oder für tert-Butyldimethylsilyloxy-Schutzgruppe stehen können,
R⁸ zusätzlich auch für eine 4-Mono-, oder 4,4'-Dimethoxytriphenylmethyloxy-Schutzgruppe stehen kann;
und Rest R⁵ auch für Trimethylsilylethinyl stehen kann.

10. Verfahren nach Anspruch 9, wobei man gegebenenfalls vorhandene 4-Mono- oder 4,4'- Dimethoxytriphenylmethyloxy Schutzgruppen gegen Hydroxyl tauscht und Acyl- und Silylreste gegebenenfalls hydrolytisch spaltet.

## Claims

1. Ethynylated heterodinucleoside phosphate analogs of formula I 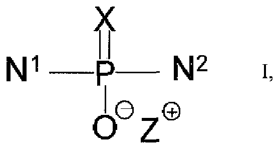 in which
X stands for 0 or S;
Z stands for H or the corresponding salt of acid addition of this compound;
N¹ stands for a nucleoside residue of formula IV 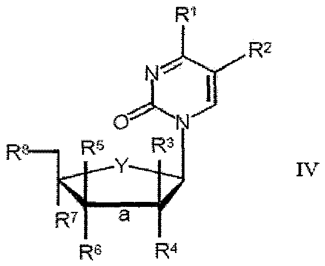 which is not ethynylated, in which:
R¹ stands for hexadecyl, palmitoyl, oleoylamino or hydroxyl;
R² stands for H or fluoro;
R³, R⁴ are identical or different and stand for H, hydroxyl, fluoro;
R⁵, R⁷ stands for H;
R⁶ stands for an oxygen atom, with which N¹ is bridged with P,
R⁸ stands for hydroxyl, azido or H and Y stands for 0 or S
and N² stands for a nucleoside residue of formula IV, which is ethynylated, in which
R¹ stands for amino
R², R³, R⁷ for H;
R⁴, R⁶ for hydroxyl;
R⁵ for ethynyl;
R⁸ stands for an oxygen atom, with which N² is bridged with P and
Y stands for 0 or S.

2. Compounds according to Claim 1, in which N² stands for a nucleoside residue of formula IV, which is ethynylated, and N¹ stands for a nucleoside residue of formula IV, which is not ethynylated, in which R¹ stands for hydroxyl;
R² stands for fluoro;
R³, R⁴, R⁵ and R⁷ stand for H,
R⁶ stands for -0-, and
R⁸ stands for hydroxyl.

3. Compound according to either of the preceding claims which is 5-fluoro-2'-deoxyuridylyl-(3'-5')-3'-C-ethynylcytidine.

4. Pharmaceutical agent, containing at least one compound according to one of the preceding claims in a pharmaceutically compatible vehicle or diluent.

5. Agent according to Claim 4, contained in liposomes or nanoparticles.

6. Agent according to Claim 4 or 5, additionally containing at least one other pharmacological active substance, which is suitable for the treatment of infectious diseases and/or cancers.

7. Use of at least one compound according to one of Claims 1 to 3 for the production of a pharmaceutical agent for the prevention and/or therapy of infectious diseases and/or cancers.

8. Method of production of ethynylated heterodinucleoside phosphate analogs according to one of Claims 1 to 3, wherein two nucleosides of general formulas Va and Vb
L¹-N¹ (Va)
L²-N² (Vb)
in which
N¹ and N² are as defined above and optionally have protecting groups; with at least one of the groups N¹ and N² on the glycosidic residue bearing an ethynyl or protected ethynyl group; and
L¹ and L² on the glycosidic residue of N¹ or N² represent bound, mutually reactive groups, one of the groups L¹ and L² standing for a hydroxy or mercapto group and the other for a hydrogenphosphonate or thiohydrogenphosphonate group, and with one of the groups L¹ and L² bound cyclically and the other bound terminally;
are condensed in the presence of an acid chloride and the condensation product is then oxidized, and optionally present protecting groups are removed.

9. Method according to Claim 8, wherein in each case two nucleosides of formulas Va and Vb are reacted, wherein the nucleosides Va and Vb correspond to a compound of the above general formula IV, in which
X and "a" have the meanings given above,
L¹ and L² are contained in place of one of the
L¹ and L² are contained in place of one of the residues R⁶ or R⁸,
the residues R¹ to R⁸ otherwise have the meaning given in Claims 1 to 7;
the residues R¹ and R³ to R⁸ additionally also stand for an acylated hydroxyl group, whose acyl residue is linear or branched, has 1-24 carbon atoms and 1 or 2 double bonds and can be substituted with an aromatic residue, or can stand for a tert-butyldimethylsilyloxy protecting group,
R⁸ additionally can also stand for a 4-mono-, or 4,4'-dimethoxytriphenylmethyloxy protecting group; and residue R⁵ can also stand for trimethylsilylethynyl.

10. Method according to Claim 9, wherein optionally present 4-mono- or 4,4'-dimethoxytriphenylmethyloxy protecting groups are exchanged for hydroxyl, and acyl and silyl residues are optionally cleaved hydrolytically.

## Revendications

1. Analogues d'hétérodinucléosides phosphates éthynylés de formule I 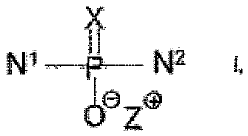 dans laquelle
X représente 0 ou S ;
Z représente H ou le sel d'addition avec un acide correspondant de ce composé ;
N¹ représente un résidu nucléosidique de formule IV 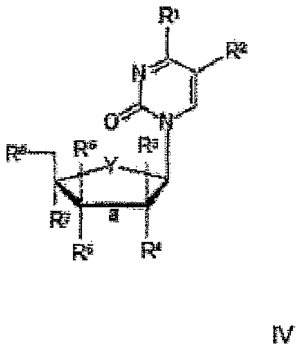 qui n'est pas éthynylé, formule dans laquelle :
R¹ représente un groupe hexadécyl-, palmitoyl-, oléoylamino ou hydroxy ;
R² représente H ou le fluor ;
R³, R⁴ sont identiques ou différents, et représentent H, hydroxy, fluor ;
R⁵, R représentent H ;
R⁶ représente un atome d'oxygène, avec lequel N¹ est relié par un pont à P,
R⁸ représente hydroxy, azido ou H et
Y représente 0 ou S
et N² représente un résidu nucléosidique de formule IV qui est éthynylé, dans lequel
R¹ représente amine,
R², R³, R représentent H ;
R⁴, R⁶ représentent hydroxy ;
R⁵ représente le groupe éthynyle ;
R⁸ représente un atome d'oxygène, avec lequel N² est relié par un pont à P et
Y représente 0 ou S.

2. Composés selon la revendication 1, dans lequel N² représente un résidu nucléosidique de formule IV qui est éthynylé, et N¹ représente un résidu nucléosidique de formule IV qui est non éthynylé, dans lequel
R¹ représente hydroxy ;
R² représente le fluor ;
R³, R⁴, R⁵ et R⁷ représentent H,
R⁶ représente -O-, et
R⁸ représente hydroxy.

3. Composé selon l'une quelconque des revendications précédentes, qui est la 5-fluoro-2'-désoxyuridylyl-(3'-5')-3'-C-éthynylcytidine.

4. Agent pharmaceutique, contenant au moins un composé selon l'une quelconque des revendications précédentes, dans un véhicule ou diluant pharmaceutiquement acceptable.

5. Agent selon la revendication 4, contenu dans des liposomes ou des nanoparticules.

6. Agent selon la revendication 4 ou 5, contenant en outre au moins une autre substance active pharmacologique qui est appropriée au traitement de maladies infectieuses et/ou de maladies cancéreuses.

7. Utilisation d'au moins un composé selon l'une quelconque des revendications 1 à 3, pour la fabrication d'un agent pharmaceutique destiné à la prévention et/ou à la thérapie de maladies infectieuses et/ou de maladies cancéreuses.

8. Procédé pour la préparation d'analogues d'hétérodinucléosides phosphates éthynylés selon l'une quelconque des revendications 1 à 3, dans lequel on condense en présence d'un chlorure d'acide deux nucléosides de formules générales Va et Vb
L¹-N¹ (Va)
L²-N² (Vb)
dans lesquelles
N¹ et N² sont tels que définis plus haut et comportent éventuellement des groupes protecteurs ; au moins l'un des groupes N¹ et N² portant sur le radical glycosidique un groupe éthynyle ou éthynyle protégé ; et
L¹ et L² représentent des groupes réactifs entre eux, liés au radical glycosidique de N ou respectivement N², l'un des groupes L¹ et L² représentant un groupe hydroxy ou mercapto et l'autre représentant un groupe hydrogénophosphate ou thiohydrogénophosphate, et l'un des groupes L¹ et L² étant lié sur le cycle et l'autre étant lié en bout de chaîne ;
et ensuite on oxyde le produit de condensation, et on élimine les groupes protecteurs éventuellement présents.

9. Procédé selon la revendication 8, dans lequel on fait réagir deux nucléosides de formules Va et Vb, les nucléosides Va et Vb correspondant à un composé de formule générale IV ci-dessus, dans lesquelles
X et a ont les significations indiquées plus haut,
L¹ et L ² sont contenus à la place d'un des radicaux R⁶ ou R⁸,
Les radicaux R¹ à R⁸ ont par ailleurs la signification indiquée dans les revendications 1 à 7 ;
les radicaux R¹ et R³ à R⁸ en outre peuvent également représenter un groupe hydroxy acylé, dont le radical acyle est linéaire ou ramifié, comporte 1-24 atomes de carbone et 1 ou 2 doubles liaisons et peut être substitué par un radical aromatique, ou peuvent représenter un groupe protecteur tert-butyldiméthylsilyloxy,
R⁸ peut en outre également représenter un groupe protecteur 4-mono- ou 4,4'-diméthoxytriphénylméthyloxy ;
et le radical R⁵ peut également représenter le groupe triméthylsilyléthynyle.

10. Procédé selon la revendication 9, dans lequel on remplace par hydroxy des groupes protecteurs 4-mono- ou 4,4'-diméthoxytriphénylméthyloxy éventuellement présents et éventuellement on sépare par hydrolyse des radicaux acyle et silyle.
